# EUROPEAN PATENT SPECIFICATION

(11) **EP 0 586 476 B1**
(45) Date of publication and mention of the grant of the patent: **27.03.1996**
(21) Application number: 92911206.8
(22) Date of filing: 27.05.1992
(51) Int. Cl.: A61K 9/14, A61K 9/16

(54) **METHOD OF PRODUCING REPRODUCIBLE PARTICLES**
VERFAHREN ZUR HERSTELLUNG VON REPRODUZIERBAREN PARTIKELN
PROCEDE DE FABRICATION DE PARTICULES REPRODUCTIBLES

(30) Priority: 27.05.1991 SE 9101603
(43) Date of publication of application: 16.03.1994
(73) Proprietor: MEDINVENT, S-756 47 Uppsala (SE)
(72) Inventor: AGERUP, Bengt, S-756 47 Uppsala (SE)
(74) Representative: Bjellman, Lennart Olov Henrik
(86) International application number: SE9200361
(87) International publication number: WO9221326

(56) References cited:
- EP-A- 0 439 373
- WO-A-89/02266

## Description

The present invention relates to a method to produce reproducable particles of defined size. More precisely, the invention relates to a method to produce aggregated drug(s) in a finely divided or atomized form.

It is known to aggregate drugs to give them an impaired solubility and, thereby, sustained or slow release in vivo. The aggregation can be achieved with a biocompatible polymer or any other substance being known to give the above properties.

The most common way to produce aggregated drug(s) is to heat these together with an aggregating agent, mix, and pour the mixture into moulds of desired size. In this way, rods and similar shapes are obtained being suitable for implantation in the body. The drawbacks with implants is that they are only produced in some standard sizes and that they require surgical insertion/removal.

Therefore, it has long been desired to be able to administrate an optional dosis of aggregated drug(s), for example by injection thereof. To enable this the aggregated drug has to be finely divided, i.e. particles have to be formed which are brought into a solution for injection.

A known way of atomization starts with mixing drug(s) and aggregating agent, heating thereof, moulding into blocks or other shapes, which thereafter are ground in special mills. The grinding and the following washing step have to be made under strict aseptic conditions to avoid, as far as possible, bacterial contamination. Besides giving poor yield, this method also requires expensive special mills to guarantee a uniform size spread.

Another way of atomization is to mix drug(s) and aggregating agent, heat the mixture and thereafter spray the same to obtain atomized "beads".

Since the above two methods rely on heating for conversion to an intermediate liquid phase, these methods also have the additional drawback that they cannot be used with heat sensible drugs, for example proteins.

A known way to obtain atomized aggregated drugs of the latter type, is to mix solvent-dissolved aggregating agent and freeze dried protein, for example hormones. However, this method gives a very low yield since pores are formed in the aggregating agent when the solvent disappears, which in turn leads to that the drug no longer is retained in the aggregating agent but is leaking out of the pores.

Yet another known way is to use a chemical pre-state of the aggregating agent during which the drug is admixed and the atomized particles are formed. Examples of this is the condition existing before a cross-linking reaction takes part.

The drawback with the known methods is that they do not provide reproducible particulate material of defined size. In other words the extent of aggregation becomes different from batch to batch, which in turn means that the release rate of the drug cannot be determined exactly.

Atomized aggregated drug(s), administratable in an optional dosis, can also be used for other purposes than injecting, for example surgical implantation or in an oral drug suspension.

To provide atomized aggregated drugs and avoid the above drawbacks, the present inventor has developped a method for production thereof, comprising the conversion of a mixture of drug(s) and aggregating agent to an intermediate liquid phase before the atomizing step.

The invention is characterized in that the liquid phase is poured on an excess of temporary matrix having a determined three-dimensional structure, in that the liquid phase is allowed to transform into solid phase at the structure phases of the matrix or in the interspaces between its structure phases, and in that the temporary matrix is removed from the solid phase thereby existing in particulate form having imprints from the structure of the temporary matrix.

According to the invention aggregated drug particles are obtained in a high yield and with a defined, reproducible size.

For injecting atomized aggregated drug(s), sizes are preferred being at the most 1 mm in diameter. Larger sized can be difficult to handle in that they occlude syringe cannulas and such at the injecting step and in that they easily fall out into in the injection solution.

The invention will now be described in more detail below in relation to some examples illustrating its performance.

### Example 1

### Atomized silicon aggregated drug(s) for injecting

Aggregating agent in the form of a silicon polymer is mixed with norgestrel hormone in desired relationship. The hormone exists in atomized form, i.e. <10 µm. To the liquid mixture a setting agent is added according to the instructions of the silicon producer. Before the silicon-hormone mixture has polymerized entirely, it is added to a temporary matrix consisting of crystalline sodium chloride being size separated to about 150 µm. Under vigorous stirring the silicon-hormone mixture is atomized in the salt crystals. When the silicon has set the sodium chloride is washed away with sterile water. Thereafter, the mixture is filtrated so that only those silicon-hormone particles having the right size remain.

### Example 2

### Atomized glycolide-aggregated drug(s) for injecting

Glycolide is heated to 160°C to obtain a liquid phase and therein a fine drug powder is mixed. The liquid glycolide mass is thereafter mixed under vigorous stirring with three to five parts temporary matrix, in this case heated sodium bicarbonate of suitably selected crystal size. When the mixture has cooled off the sodium bicarbonate is washed away with sterile water, whereafter possible large fragments are filtrated away so that only aggregated particles having a uniform size are obtained. When using large bicarbonate crystals large particles are obtained while small give rise to small particles.

The yield in this example was 90% particles of the right size, which is to be compared with block moulding followed by grinding which only gives about 30% particles of the right size.

### Example 3

### Drug(s) enclosed in fragment of cross-linked connective tissue matrix for local application in association with surgical operations

To 200 ml of 0.5% solution of hyaluronan the amount giving the required activity of the growth factor IGF (insulin-like growth factor) is added. Under vigorous stirring 250 mg divinylsulphone and 1 ml 0.5 M NaOH is added. The resulting mixture is poured under stirring onto a temporary matrix, in this case 400 g size separated aluminiumhydroxide. When the material is fully admixed with the matrix, it is allowed to stand for 1 hour. The resulting cross-linked gel matrix is separated by adding an excess of saline. The upper phase is kept and washed over a fine mesh filtre while heavier aluminiumhydroxide is washed to be reused. The thus produced drug gel comprises small fragments in which the enclosed growth factor has been captured. These fragments have a "closed" form and release growth factor with a controlled rate. The yield in this Example was about 80%.

Previously, gel fragments have been obtained by pressing the gel through a fine mesh net. These have an "open" form and, therefore, release drug(s) in an uncontrollable way.

### Example 4

### Removal of nauseating taste of an oral pediatric antibiotic suspension

4 g finely ground metronidazol were mixed with 4 g Eudragit® (an acrylate formulation) dissolved in chloroform. The mixture is brought into a temporary matrix, in this case 50 g of flavoured dry fructose powder. After drying and removal of the remaining fructose powder a tasty microgranulate is obtained being easy to swallow in liquid without the nauseating taste of the active component.

Besides obtaining the atomized aggregated drugs according to the invention in constantly higher yield in the above described examples 1-4, a number of other advantages are obtained.

By using crystals, microspheres, fibres or other temporary matrix, being size determined and having a known structure, it is possible to obtain aggregated drug(s) having exactly the same degree of atomization. At the point of injecting, this means that the release rate and -retention time of the drug in vivo can be determined exactly. There are small losses because only few fragments differ from the desired size and the losses during washing and such are neglectible, giving a high yield with accompanying economic advantages.

The temporary matrix can be of reusable or disposable type, examples of the former are difficultly soluble salts, such as the above mentioned aluminiumhydroxide, microspheres, for example polystyrene beads, and fibres.

Exemples of in vivo decomposible biocompatible polymers for aggregating are, for example, polyglycolide (see Example 2) and co-polymers thereof, polylactides and co-polymers thereof, poly-β-hydroxybutyrate and co-polymerisates thereof, poly-p-dioxanon, poly-δ-valerolactone, poly-ε-caprolactone, methyl-metacrylate-N-vinyl pyrrolidine co-polymers, polyesther amides, polyesters of oxalic acid, polydihydropyranes, polyalcyl-2-cyanoacrylates, polyurethanes, polyvinylalcohol, polypeptides, poly-β-maleicacid and poly-β-alcanoic acids.

Examples of stabile biocompatible polymeres that can be used according to the invention are silicon (see Example 1) and Teflon®, among others being known in the art.

To avoid losses at the injecting step, it is suitable to use a combination of aggregated drug(s) and a pseudoplastic gel, such as is described in SE-C-465950.

The present invention also offers opportunity to vary the dosis of aggregated drug(s) for implantation giving, at the same time, a controlled release rate in vivo. Furthermore, the aggregated drug is surrounded by a biocompatible protection in vivo, i.e. by the hyaluronic acid (see Example 3 above).

All the steps in the production methods according to the invention are performed in a closed system, for example a centrifugal drum, leading to a minimum of bacterial contamination.

It is possible to "track" use of the method according to the invention because the temporary matrix is leaving characterizing impressions in the surfaces of the atomized aggregated drugs, the impressions appearing clearly in a microscope, for example impressions caused by a special type of crystal.

## Claims

1. A method for producing atomized aggregated drug(s), comprising conversion of a mixture of drug(s) and aggregating agent to an intermediate liquid phase before an atomization step, **characterized in** that said liquid phase is poured onto an excess of temporary matrix having a determined three-dimensional structure, in that said liquid phase is allowed to convert to a solid phase at the structure faces of the matrix or in the interspaces between its structure faces and in that said temporary matrix is removed from said solid phase which thereby exists in a particulate form having impressions from the structure of said temporary matrix.

2. A method according to claim 1, **characterized in** that said liquid phase is obtained by heating a difficultly soluble aggregating agent and drug(s), said liquid phase is allowed to set on water-soluble crystals, and in that said water-soluble crystals are washed away with sterile water.

3. A method according to claim 2, **characterized in** that said aggregating agent is biocompatible polymer.

4. A method according to claim 3, **characterized in** that said aggregating agent is an in vivo decomposible biocompatible polymer, selected from the group consisting of polyglycolide and co-polymers thereof, polylactides and co-polymers thereof, poly-β-hydroxybutyrate and co-polymers thereof, poly-p-dioxanone, poly-δ-valerolactone, poly-ε-caprolactone, methyl-metacrylate-N-vinyl pyrrolidine co-polymers, polyesther amides, polyesters of oxalic acid, polydihydropyranes, polyalcyl-2-cyanoacrylates, polyurethanes, polyvinylalcohol, polypeptides, poly-β-maleic acid and poly-β-alcanoic acids.

5. A method according to claim 1, **characterized in** that said liquid phase consists of drug(s) and unpolymerized aggregating agent, which after addition of a setting agent are able to polymerize on and about a temporary matrix of water soluble crystals, and in that said water-soluble crystals are washed away with sterile water.

6. A method according to claim 5, **characterized in** that said aggregating agent is a stabile biocompatible polymer, for example silicon or polytetrafluorethene.

7. A method according to claim 2 or 5, **characterized in** that said temporary matrix is sodium chloride or sodium bicarbonate.

8. A method according to claim 1, **characterized in** that said liquid phase consists of drug(s) and unpolymerized aggregating agent, which after addition of a cross-linking agent are able to polymerize on and about a temporary matrix of difficultly soluble crystals, and in that said polymerized phase is separated from said difficultly soluble crystals, whereby an excess of saline is added and said polymerized phase is obtained in the upper phase.

9. A method according to claim 8, **characterized in** that said aggregating agent is cross-linked hyalurane, and in that said temporary matrix is aluminiumhydroxide.

10. A method according to claim 1, **characterized in** that said liquid phase is obtained in that said aggregating agent is dissolved in a volatile solvent and mixed with drug(s), in that said liquid phase is allowed to dry on and about temporary matrix, and in that the obtained atomized aggregates are separated from said matrix by sieving.

11. A method according to claim 10, **characterized in** that said temporary matrix is a fine powder.

12. A method according to claim 1, **characterized in** that said temporary phase comprises microspheres.

13. A method according claim 1, **characterized in** that said temporary phase comprises fibres.

14. Atomized aggregated drug(s) producable by the method according to one or more of the claims 1-13.

## Patentansprüche

1. Verfahren zur Herstellung von fein verteiltem(n) aggregiertem(n) Arzneimittel(n), umfassend die Umwandlung einer Mischung von Arzneimittel(n) und Aggregierungsmittel in eine intermediäre Flüssigphase vor dem Verteilungsschritt, dadurch gekennzeichnet, daß die flüssige Phase auf einen Überschuß einer temporären Matrix gegeben wird, welche eine vorbestimmte dreidimensionale Struktur aufweist, und daß die flüssige Phase auf den Strukturoberflächen oder in den Zwischenräumen zwischen den Strukturoberflächen der Matrix in eine feste Phase umwandeln gelassen wird, und daß die temporäre Matrix von der festen Phase entfernt wird, welche dabei in Teilchenform vorliegt und Abdrücke von der Struktur der temporären Matrix aufweist.

2. Verfahren gemäß Anspruch 1, dadurch gekennzeicnnet, daß die flüssige Phase durch Erhitzen eines schwerlöslichen Aggregierungsmittels und Arzneimittel(n) erhalten wird, daß man die flüssige Phase auf wasserlöslichen Kristallen absetzen läßt und daß die wasserlöslichen Kristalle mit sterilem Wasser ausgewaschen werden.

3. Verfahren gemäß Anspruch 2, dadurch gekennzeichnet, daß das Aggregierungsmittel biokompatibles Polymer ist.

4. Verfahren gemäß Anspruch 3, dadurch gekennzeichnet, daß das Aggregierungsmittel ein in vivo zersetzbares biokompatibles Polymer ist, ausgewählt aus der Gruppe, bestehend aus Polyglycolid und Co-polymeren hiervon, Polylactiden und Co-polymeren hiervon, Poly-β-hydroxybutyrat und Co-polymeren hiervon, Poly-p-dioxanon, Poly-δ-valerolacton, Poly-ε-caprolacton, Methyl-methacrylat-N-vinylpyrrolidin-Co-polymeren, Polyesteramiden, Polyestern von Oxalsäure, Polydihydropyranen, Polyalcyl-2-cyanoacrylaten, Polyurethanen, Polyvinylalkohol, Polypeptiden, Poly-β-maleinsäure und Poly-β-alkansäuren.

5. Verfahren gemäß Anspruch 1, dadurch gekennzeichnet, daß die flüssige Phase aus Arzneimittel(n) und unpolymerisierbarem Aggregierungsmittel besteht, die nach der Zugabe eines Niederschlagmittels auf und um die temporäre Matrix der wasserlöslichen Kristalle polymerisieren können und daß die wasserlöslichen Kristalle mit sterilem Wasser ausgewaschen werden.

6. Verfahren gemäß Anspruch 5, dadurch gekennzeichnet, daß das Aggregierungsmittel ein stabiles biokompatibles Polymer, beispielsweise Silicon oder Polytetrafluorethen, ist.

7. Verfahren nach Anspruch 2 oder 5, dadurch gekennzeichnet, daß die temporäre Matrix Natriumchlorid oder Natriumbicarbonat ist.

8. Verfahren gemäß Anspruch 1, dadurch gekennzeichnet, daß die flüssige Phase aus Arzneimittel(n) und unpolymerisiertem Aggregierungsmittel besteht, welche nach Zugabe eines Quervernetzungsmittels auf und um die temporäre Matrix der schwerlöslichen Kristalle polymerisieren können, und daß die polymerisierte Phase von den schwerlöslichen Kristallen getrennt wird, wobei ein Überschuß an Saline hinzugefügt wird, und daß die polymerisierte Phase in der oberen Phase erhalten wird.

9. Verfahren gemäß Anspruch 8, dadurch gekennzeichnet, daß das Aggregierungsmittel quervernetztes Hyaluran und die temporäre Matrix Aluminiumhydroxid ist.

10. Verfahren gemäß Anspruch 1, dadurch gekennzeichnet, daß die flüssige Phase dadurch erhalten wird, daß das Aggregierungsmittel in einem flüchtigen Lösungsmittel gelöst und mit Arzneimittel(n) gemischt wird, daß die flüssige Phase auf und um die temporäre Matrix trocknen gelassen wird und daß die erhaltenen fein verteilten Aggregate durch Sieben von der Matrix getrennt werden.

11. Verfahren gemäß Anspruch 10, dadurch gekennzeichnet, daß die temporäre Matrix ein feines Pulver ist.

12. Verfahren gemäß Anspruch 1, dadurch gekennzeichnet, daß die temporäre Phase Mikrosphären umfaßt.

13. Verfahren gemäß Anspruch 1, dadurch gekennzeichnet, daß die temporäre Phase Fasern umfaßt.

14. Feinverteilte(s) aggregierte(s) Arzneimittel, herstellbar nach der Methode gemäß einem oder mehreren der Ansprüche 1-13.

## Revendications

1. Un procédé de fabrication de médicament(s) pulvérisé(s) agrégé(s), comprenant la conversion d'un mélange de médicament(s) et d'un agent d'agrégation en une phase liquide intermédiaire avant l'étape de pulvérisation, caractérisé en ce que ladite phase liquide est versée dans un excès de matrice provisoire présentant une structure tridimensionnelle déterminée, en ce que on laisse ladite phase liquide se transformer en une phase solide au niveau des faces de la structure de la matrice ou au niveau des espaces situés entre les faces de sa structure et en ce que ladite matrice provisoire est retirée de ladite phase solide qui se retrouve ainsi sous une forme particulaire ayant des impressions de la structure de ladite matrice provisoire.

2. Un procédé selon la revendication 1, caractérisé en ce que ladite phase liquide est obtenue par chauffage d'un agent d'agrégation difficilement soluble et de médicament(s), en ce qu'on laisse ladite phase liquide se déposer sur les cristaux hydrosolubles et en ce que les cristaux hydrosolubles sont éliminés par lavage avec de l'eau stérile.

3. Un procédé selon la revendication 2, caractérisé en ce que ledit agent d'agrégation est un polymère biocompatible.

4. Un procédé selon la revendication 3, caractérisé en ce que ledit agent d'agrégation est un polymère biocompatible susceptible de se décomposer in vivo, sélectionné dans le groupe consistant en polyglycolide et les copolymères en dérivant, les polylactides et les copolymères en dérivant, le poly-β-hydroxybutyrate et les copolymères en dérivant, le poly-p-dioxanone, le poly-δ-valérolactone, le poly-ε-caprolactone, les copolymères de métacrylate de méthyle-N-vinylpyrrolidine, les polyesteramides, les polyesters d'acide oxalique, les polydihydropyranes, les polyalcyl-2-cyanoacrylates, les polyuréthanes, le polyvinylalcool, les polypeptides, le polyacide-β-maléique et les polyacides-β-alcanoïques.

5. Un procédé selon la revendication 1, caractérisé en ce que ladite phase liquide consiste en un ou plusieurs médicaments et en un agent d'agrégation non polymérisé, qui, après addition d'un agent de sédimentation, sont capables de se polymériser sur et autour d'une matrice provisoire de cristaux hydrosolubles, et en ce que lesdits cristaux hydrosolubles sont éliminés par lavage avec de l'eau stérile.

6. Un procédé selon la revendication 5, caractérisé en ce que ledit agent d'agrégation est un polymère biocompatible stable, par exemple un silicone ou du polytétrafluoréthène.

7. Un procédé selon la revendication 2 ou 5, caractérisé en ce que ladite matrice provisoire est du chlorure de sodium ou du bicarbonate de sodium.

8. Un procédé selon la revendication 1, caractérisé en ce que ladite phase liquide consiste en un ou plusieurs médicaments et un agent d'agrégation non polymérisé, qui, après addition d'un agent de réticulation, sont capables de se polymériser sur et autour d'une matrice provisoire de cristaux difficilement solubles, et en ce que ladite phase polymérisée est séparée desdits cristaux difficilement solubles, dans lequel un excès de sel est ajouté et ladite phase polymérisée est obtenue dans la phase supérieure.

9. Un procédé selon la revendication 8, caractérisé en ce que ledit agent d'agrégation est du hyalurane réticulé et en ce que ladite matrice provisoire est de l'hydroxyde d'aluminium.

10. Un procédé selon la revendication 1, caractérisé en ce que ladite phase liquide est obtenue, en ce que ledit agent d'agrégation est dissous dans un solvant volatil et mélangé avec le ou les médicaments, en ce que on laisse ladite phase liquide sécher sur et autour de la matrice provisoire, et en ce que les agrégats pulvérisés obtenus sont séparés de ladite matrice par tamisage.

11. Un procédé selon la revendication 10, caractérisé en ce que ladite matrice provisoire est une poudre fine.

12. Un procédé selon la revendication 1, caractérisé en ce que ladite phase provisoire comprend des microsphères.

13. Un procédé selon la revendication 1, caractérisé en ce que ladite phase provisoire comprend des fibres.

14. Médicament(s) pulvérisé(s) agrégé(s) susceptible(s) d'être obtenu(s) par le procédé selon l'une quelconque des revendications 1 à 13.
